# EUROPÄISCHE PATENTSCHRIFT

(11) **EP 1 027 331 B1**
(45) Veröffentlichungstag und Bekanntmachung des Hinweises auf die Patenterteilung: **09.10.2002**
(21) Anmeldenummer: 98955433.2
(22) Anmeldetag: 12.10.1998
(51) Int. Cl.: C07C 319/06, C07C 321/26

(54) **VERFAHREN ZUR HERSTELLUNG VON ARYLMERCAPTANEN DURCH HYDRIERUNG VON DIARYLDISULFIDEN**
METHOD FOR PRODUCING ARYL THIOLS BY HYDROGENATING DIARYLDISULPHIDES
PROCEDE DE PREPARATION DE THIOLS D'ARYLE PAR HYDROGENATION DE DIARYLDISULFURES

(30) Priorität: 22.10.1997 DE 19746512
(43) Veröffentlichungstag der Anmeldung: 16.08.2000
(73) Patentinhaber: Bayer Aktiengesellschaft, 51368 Leverkusen (DE)
(72) Erfinder: ULLRICH, Friedrich-Wilhelm, D-51465 Bergisch Gladbach (DE); FIEGE, Helmut, D-51373 Leverkusen (DE)
(86) Internationale Anmeldenummer: EP9806450
(87) Internationale Veröffentlichungsnummer: WO99020602

(56) Entgegenhaltungen:
- DE-A- 1 768 421
- PATENT ABSTRACTS OF JAPAN vol. 005, no. 148 (C-072), 18. September 1981 & JP 56 081541 A (MITSUI PETROCHEMICAL INDUSTRIES), 3. Juli 1981
- PATENT ABSTRACTS OF JAPAN vol. 010, no. 056 (C-331), 6. März 1986 & JP 60 199871 A (NIPPON KAYAKU), 9. Oktober 1985 in der Anmeldung erwähnt

## Beschreibung

Die vorliegende Erfindung betrifft ein besonders vorteilhaftes Verfahren zur Herstellung von Arylmercaptanen durch Hydrierung von Diaryldisulfiden.

Arylmercaptane sind wichtige Zwischenprodukte zur Herstellung von pharmazeutischen und agrochemischen Wirkstoffen (siehe z.B. EP-A 100 172, BE-A 887 423, US-A 3 912 757 und WO 96/25936).

Ein brauchbares Verfahren zur Herstellung von Arylmercaptanen besteht in der Reaktionssequenz der Reduktion von Arylsulfochloriden mit Natriumsulfit zu Arylsulfinsäuresalzen, weiterer Reduktion mit schwefliger Säure zum Diaryldisulfid und schließlich Spaltung des Disulfids mit Natriumborhydrid (DE-A 44 20 777). Unbefriedigend ist hier die Spaltung des Disulfids mit Natriumborhydrid, die zwar in guter Ausbeute verläuft, jedoch mit dem Natriumborhydrid ein nur aufwendig herzustellendes und deshalb teures Reagenz benötigt.

Katalytische hydrierende Spaltungen von Diaryldisulfiden sind auch beschrieben, sie verlaufen aber stets bei hohen Temperaturen und Drucken oder erfordern sonstige nachteilige Maßnahmen. So wird gemäß J. Org. Chem. 24, 1598 (1959) in Toluol mit einem MoS₂-Aluminiumoxid-Katalysator bei 140°C und 124 bar gearbeitet. In der DE-A 17 68 421 wird diese Hydrierung mit Palladium-Kontakten und Raney-Cobalt bei 160 bis 200°C und 150 bar beschrieben. Gemäß der JP-OS 60/199 871 wird die Hydrierung mit Raney-Nickel in einem heterogenen Lösungsmittelsystem durchgeführt, das aus zwei Phasen besteht, nämlich einer wäßrig/alkalischen und einer wasserunlöslichen organischen Phase.

Wenn man bei diesen Verfahren zu niedrigeren Drucken und niedrigeren Temperaturen übergeht stellt sich ein nur unvollständiger Umsatz ein (siehe Vergleichsbeispiele 1 und 2).

Es wurde nun ein Verfahren zur Herstellung von Arylmercaptanen durch katalytische Hydrierung von Diaryldisulfiden gefunden, das dadurch gekennzeichnet ist, daß man die Hydrierung in einem basischen alkoholischen Medium durchführt.

Nach dem erfindungsgemäßen Verfahren kann man beispielsweise Arylmercaptane der Formel (I) 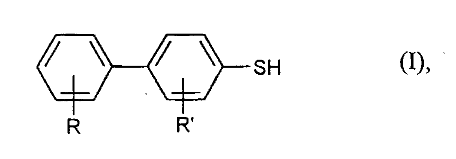 in der
- R und R': unabhängig voneinander jeweils für Wasserstoff, C₁-C₆-Alkyl, C₁-C₆-Alkoxy oder Halogen stehen,
aus den entsprechenden Diaryldisulfiden der Formel (II) herstellen 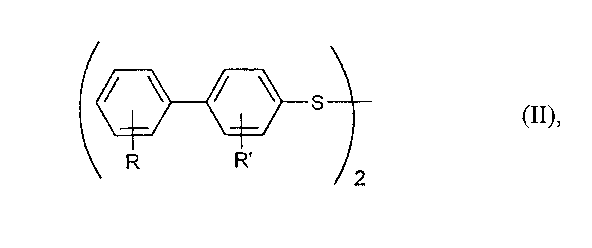 in der
- R und R': die bei Formel (I) angegebene Bedeutung haben.

In den Formeln (I) und (II) stehen R und R' unabhängig voneinander vorzugsweise für Wasserstoff, Methyl, Ethyl, Methoxy, Ethoxy, Fluor oder Chlor. Besonders bevorzugt stehen R und R' für Wasserstoff.

Die als Ausgangsprodukt benötigten Diaryldisulfide, insbesondere solche der Formel (II), sind auf verschiedene Weise zugänglich, z.B. gemäß der DE-A 44 20 777. Insbesondere ist hier ein Verfahren von Interesse, bei dem man S-(4-Biphenyl)-sulfinsäure oder deren Salze mit einer wäßrigen Bisulfitlösung bei pH-Werten von 2 bis 7 umsetzt, so die entsprechenden S-(4-Biphenyl)-thioschwefelsäuren oder deren Salze erhält und diese in Gegenwart einer starken wäßrigen Säure erhitzt. Dieses Verfahren ist ausführlicher in einer anderen Patentanmeldung beschrieben, die gleichzeitig vom gleichen Anmelder eingereicht wurde.

Als Lösungsmittel zur Bildung des erfindungsgemäß erforderlichen alkoholischen Mediums sind z.B. aliphatische C₁-C₅-Alkohole geeignet, die geradkettig oder verzweigt sein können. Es kann sich dabei um primäre, sekundäre oder tertiäre Alkohole handeln. Bevorzugt sind Methanol, Ethanol und Isopropanol. Das alkoholische Medium kann gegebenenfalls auch Wasser enthalten, beispielsweise bis zu 20 Gew.-%, vorzugsweise bis zu 10 Gew.-%. So ist es beispielsweise nicht erforderlich, das Diaryldisulfid in getrockneter Form einzusetzen. Man kann es genausogut in Form des bei seiner Herstellung anfallenden wasserfeuchten Produktes einsetzen. Man kann als alkoholisches Medium auch Gemische verschiedener Alkohole verwenden.

Bezogen auf 1 Mol Diaryldisulfid kann man z.B. 200 bis 10.000 ml Alkohol einsetzen. Vorzugsweise liegt diese Menge bei 500 bis 5.000 ml.

Als Mittel zur Erzeugung eines basischen Charakters im alkoholischen Medium können z.B. Alkalihydroxide, Alkalicarbonate oder Alkalialkoholate, insbesondere Alkali-C₁-C₄-alkylalkoholate oder entsprechende Erdalkaliverbindungen eingesetzt werden. Bevorzugt sind Alkalihydroxide, insbesondere Natrium- und Kaliumhydroxid.

Bezogen auf 1 Mol Diaryldisulfid kann man beispielsweise 0,5 bis 5 Äquivalente Base einsetzen. Bevorzugt liegt diese Menge bei 1,5 bis 2,5 Äquivalenten.

Als Katalysatoren für die erfindungsgemäße Hydrierung kommen z.B. Metalle der 8. Nebengruppe des PSE in Frage, insbesondere Nickel und Palladium. Derartige Metalle können als solche oder als Metallverbindungen eingesetzt werden. Metalle oder Metallverbindungen können gegebenenfalls auf Trägermaterialien wie Aktivkohle, Aluminiumoxid, Erdalkalicarbonat oder Erdalkalisulfat angeordnet sein. Die Katalysatoren können mit weiteren Metallen oder Metallverbindungen dotiert sein, beispielsweise solchen aus der 4. oder 8. Nebengruppe des PSE. Sie können auch in Form von Skelettkatalysatoren vorliegen, z.B. in Raney-Form. Bevorzugte Katalysatoren sind Palladium-auf-Kohle, Palladium-Schwarz, Raney-Nickel und mit Cobalt und/oder Eisen dotiertes Raney-Nickel. Besonders bevorzugt ist Raney-Nickel.

Die erfindungsgemäße katalytische Hydrierung kann z.B. bei Temperaturen von 20 bis 200°C und Drucken bis zu 50 bar durchgeführt werden. Bevorzugt sind Temperaturen von 40 bis 150°C und Drucke bis zu 25 bar, insbesondere Temperaturen von 60 bis 120°C und Drucke bis zu 15 bar.

Die Aufarbeitung des nach der erfindungsgemäßen Hydrierung vorliegenden Reaktionsgemisches kann z.B. so erfolgen, daß man es abkühlt, beispielsweise auf 20 bis 65°C, den Katalysator abtrennt, z.B. durch Filtration, die dann vorliegende alkoholische Lösung des hergestellten Arylmercaptans mit Säure und gegebenenfalls Wasser versetzt und das dann ausfallende Produkt abfiltriert und trocknet.

Mit dem erfindungsgemäßen Verfahren kann man Arylmercaptane, insbesondere Diarylmercaptane der Formel (I) in guten Ausbeuten und Reinheiten erhalten.

Das erfindungsgemäße Verfahren hat überraschende Vorteile. So benötigt es keine aufwendig herzustellenden Reagenzien. Es kann bei niedrigeren Temperaturen und insbesondere bei niedrigeren Drucken als andere Verfahren durchgeführt werden, was geringeren Aufwand an Apparaturen und Handling bedeutet. Schließlich vermeidet es eine umständliche Arbeitsweise mit zwei nebeneinander vorliegenden flüssigen Phasen. Überraschend ist dabei insbesondere die Tatsache, daß aus Houben-Weyl, Methoden der organischen Chemie, Band IX, S. 77 (1955) in basischem Medium die Disproportionierung von Disulfiden zu Sulfinaten und Thiolen bekannt ist. Diese unerwünschte Reaktion hat man bisher durch den Einsatz von mit Wasser nicht mischbaren Lösungsmitteln zu unterdrücken versucht. Überraschenderweise hat sich gezeigt, daß dies nicht notwendig ist.

### Beispiele

### Beispiel 1

In einem 0,3 l Edelstahl-Autoklaven wurden 0,1 Mol Bis-(4-diphenyl)-disulfid in einer Lösung von 8 g Natriumhydroxid in 220 g Ethanol suspendiert und mit 1 g Raney-Nickel versetzt. Der Ansatz wurde bei 80°C mit 10 bar Wasserstoff hydriert. Nach Beendigung der Wasserstoffaufnahme wurde abgekühlt und entspannt. Nach dem Abfiltrieren des Katalysators bei 60°C wurde mit 150 ml Wasser verdünnt und mit 20 ml wäßriger Salzsäure (37 gew.-%ig) ein pH-Wert von 1 eingestellt. Man saugte das ausgefallene Produkt ab und wusch mit Wasser. Nach dem Trocknen erhielt man 35,1 g 4-Mercaptodiphenyl mit einem Gehalt von 95,7 Gew.-%. Dies entspricht einer Ausbeute von 90,2 % d.Th.

### Vergleichsbeispiel 1

Die Hydrierung wurde wie in Beispiel 1 durchgerührt, jedoch ohne Zusatz von Natriumhydroxid. Es wurde kein Wasserstoff aufgenommen. Bei der Aufarbeitung des Reaktionsgemisches wurde nur das Einsatzmaterial zurückerhalten.

### Vergleichsbeispiel 2 (analog JP-OS 60/199 871)

In einem Zweiphasen-System aus 100 ml Toluol und einer Lösung von 8 g Natriumhydroxid in 150 ml Wasser wurden 38,4 g Bis-(4-diphenyl)-disulfid suspendiert. Man versetzte mit 1 g Raney-Nickel und hydrierte den Ansatz bei 80°C und 10 bar. Nach 4 Stunden kam die Wasserstoffaufnahme zum Stillstand. Man kühlte ab und entspannte den Ansatz.

Das Reaktionsgemisch enthielt außer dem Katalysator noch unumgesetztes Bis-(4-diphenyl)-disulfid als Feststoff. In der Toluolphase wurde nach Einengen ebenfalls noch Bis-(4-diphenyl)-disulfid wiedergefunden. Die Wasserphase wurde mit 37 gew.-%iger wäßriger Salzsäure angesäuert, das ausfallende Produkt abgesaugt und mit 100 ml Wasser gewaschen. Nach dem Trocknen wurden 21,4 g 4-Mercaptodiphenyl mit einem Gehalt von 93,6 Gew.-% erhalten. Das entspricht einer Ausbeute von 53,8 % d.Th.

### Beispiel 2

Die Hydrierung wurde wie in Beispiel 1 durchgeführt, jedoch wurde Methanol an Stelle von Ethanol als Lösungsmittel eingesetzt. Man erhielt 37,1 g 4-Mercaptodiphenyl mit einem Gehalt von 97,1 Gew.-%. Das entsprach einer Ausbeute von 96,7 % d.Th.

### Beispiel 3

Die Hydrierung wurde wie in Beispiel 1 durchgeführt, jedoch wurde Isopropanol an Stelle von Ethanol als Lösungsmittel eingesetzt. Man erhielt 31,8 g 4-Mercaptodiphenyl mit einem Gehalt von 81,7 Gew.-%. Das entsprach einer Ausbeute von 69,8 % d.Th.

### Beispiel 4

189 g Bis-(4-diphenyl)-disulfid wurde wie in Beispiel 5 beschrieben hergestellt, jedoch in Form des feuchten Nutschkuchens (276 g) in die Hydrierung eingesetzt. Dazu wurde der feuchte Nutschkuchen mit 800 g Ethanol angeschlämmt, mit 44 g Natriumhydroxid und 5,5 g Raney-Nickel versetzt. Die Mischung erwärmte man in einem Autoklaven auf 80°C und drückte 10 bar Wasserstoff auf. Die Wasserstoffaufnahme war nach 4 Stunden beendet. Der Ansatz wurde abgekühlt und entspannt. Nach Abfiltrieren des Katalysators bei 60°C wurde das Filtrat mit 840 ml 5,4 gew.-%iger wäßriger Salzsäure angesäuert. Dabei fiel das Produkt aus. Es wurde abgesaugt, mit 250 ml Wasser gewaschen und getrocknet. Man erhielt 178,8 g eines weißen Pulvers mit dem Schmelzpunkt von 110 bis 112°C. Der Gehalt an 4-Mercaptodiphenyl lag bei 91,8 Gew.-% (iodometrisch bestimmt). Daraus ergibt sich eine Ausbeute von 80,1 % d.Th. bezogen auf ursprünglich eingesetztes Diphenyl-4-sulfochlorid.

### Beispiel 5 (Herstellung von Bis-(4-diphenyl)-disulfid- nicht erfindungsgemäß)

400 ml 39 gew.-%ige wäßrige Bisulfitlauge und 550 ml Wasser wurden mit 125 ml wäßriger Natronlauge (45 gew.-%ig) vorgelegt und mit 2,2 g Triethylbenzylammoniumchlorid versetzt. Die Mischung wurde auf 60°C erwärmt. Im Verlauf einer Stunde wurden 289,5 g Diphenyl-4-sulfochlorid (96 gew.-%ig) eingetragen und gleichzeitig der pH-Wert des Reaktionsgemisches durch Zudosierung von 45 gew.-%iger wäßriger Natronlauge auf 8 gehalten.Auch während der dreistündigen Nachrührzeit wurde der pH-Wert durch Natronlauge-Dosierung konstant gehalten. Die Reaktionsmischung wurde dann in einem 3 l Email-Autoklaven mit 80 ml Schwefeldioxid versetzt und 3 Stunden lang auf 130°C erhitzt. 6 Stunden wurde der Ansatz bei 130°C und einem Druck von 4,4 bis 6,9 bar gerührt. Anschließend kühlte man auf Raumtemperatur und entspannte. Die erhaltene Suspension wurde abgesaugt. Der Nutschkuchen wurde getrocknet. Man erhielt 237,4 g Feststoff mit einem Gehalt an Bis-(4-diphenyl)-disulfid von 78,3 %. Das entspricht einer Ausbeute von 91,2 % d.Th.

## Patentansprüche

1. Verfahren zur Herstellung von Arylmercaptanen durch katalytische Hydrierung von Diaryldisulfiden, **dadurch gekennzeichnet, daß** man die Hydrierung in einem basischen alkoholischen Medium durchführt.

2. Verfahren nach Anspruch 1, **dadurch gekennzeichnet, daß** man Arylmercaptane der Formel (I) 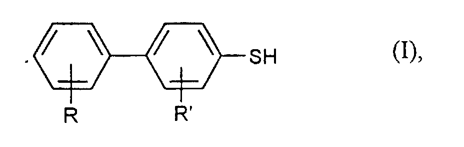 in der
R und R' unabhängig voneinander jeweils für Wasserstoff, C₁-C₆-Alkyl, C₁-C₆-Alkoxy oder Halogen stehen,
aus den entsprechenden Diaryldisulfiden der Formel (II) herstellt 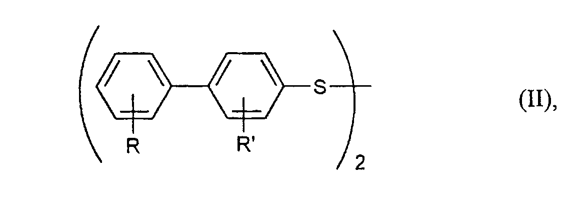 in der
R und R' die bei Formel (I) angegebene Bedeutung haben.

3. Verfahren nach Ansprüchen 1 und 2, **dadurch gekennzeichnet, daß** man als Lösungsmittel zur Bildung des alkoholischen Mediums aliphatische C₁-C₅-Alkohole einsetzt.

4. Verfahren nach Ansprüchen 1 bis 3, **dadurch gekennzeichnet, daß** das alkoholische Medium bis zu 20 Gew.-% Wasser enthält.

5. Verfahren nach Ansprüchen 1 bis 4, **dadurch gekennzeichnet, daß** man als Mittel zur Erzeugung eines basischen Charakters im alkoholischen Medium Alkalihydroxide, Alkalicarbonate oder Alkalialkoholate oder entsprechende Erdalkaliverbindungen einsetzt.

6. Verfahren nach Ansprüchen 1 bis 5, **dadurch gekennzeichnet, daß** man auf I Mol Diaryldisulfid 0,5 bis 5 Äquivalente Base einsetzt.

7. Verfahren nach Ansprüchen 1 bis 6, **dadurch gekennzeichnet, daß** man als Katalysatoren Metalle der 8. Nebengruppe des PSE einsetzt.

8. Verfahren nach Ansprüchen 1 bis 7, **dadurch gekennzeichnet, daß** man die katalytische Hydrierung bei Temperaturen von 20 bis 200°C und Drucken bis zu 50 bar durchführt.

9. Verfahren nach Ansprüchen 1 bis 8, **dadurch gekennzeichnet, daß** man die katalytische Hydrierung bei 40 bis 150°C und Drucken bis zu 25 bar durchführt.

10. Verfahren nach Ansprüchen 1 bis 9, **dadurch gekennzeichnet, daß** man das nach Durchführung der katalytischen Hydrierung vorliegende Reaktionsgemisch aufarbeitet, indem man es abkühlt, den Katalysator abtrennt, die dann vorliegende alkoholische Lösung des hergestellten Arylmercaptans mit Säure versetzt und das dann ausfallende Produkt abfiltriert und trocknet.

## Claims

1. Process for preparing aryl mercaptans by catalytic hydrogenation of diaryl disulphides, **characterized in that** the hydrogenation is carried out in a basic alcoholic medium.

2. Process according to Claim 1, **characterized in that** aryl mercaptans of the formula (I) 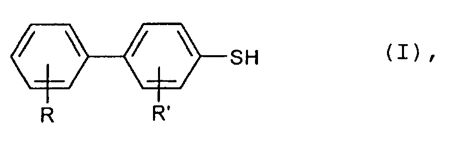 in which
R and R' independently of one another each represent hydrogen, C₁-C₆-alkyl, C₁-C₆-alkoxy or halogen,
are prepared from the corresponding diaryl disulphides of the formula (II) 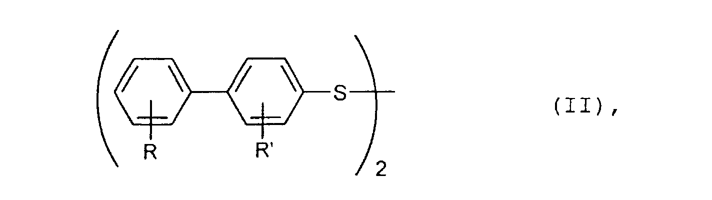 in which
R and R' are as defined in formula (I).

3. Process according to Claims 1 and 2, **characterized in that** the solvents used for forming the alcoholic medium are aliphatic C₁-C₅-alcohols.

4. Process according to Claims 1 to 3, **characterized in that** the alcoholic medium comprises up to 20% by weight of water.

5. Process according to Claims 1 to 4, **characterized in that** the agents used for generating a basic character in the alcoholic medium are alkali metal hydroxides, alkali metal carbonates or alkali metal alkoxides or the corresponding alkaline earth metal compounds.

6. Process according to Claims 1 to 5, **characterized in that** 0.5 to 5 equivalents of base are employed per mole of diaryl disulphide.

7. Process according to Claims 1 to 6, **characterized in that** the catalysts used are metals of the 8^{th} transition group of the PTE.

8. Process according to Claims 1 to 7, **characterized in that** the catalytic hydrogenation is carried out at temperatures of from 20 to 200°C and pressures of up to 50 bar.

9. Process according to Claims 1 to 8, **characterized in that** the catalytic hydrogenation is carried out at 40 to 150°C and pressures of up to 25 bar.

10. Process according to Claims 1 to 9, **characterized in that** the reaction mixture which is present after the catalytic hydrogenation has been carried out is worked up **in that** it is cooled, the catalyst is separated off, the resulting alcoholic solution of the aryl mercaptan which has been prepared is treated with acid and the precipitated product is filtered off and dried.

## Revendications

1. Procédé pour la préparation d'arylmercaptans par hydrogénation catalytique de diaryldisulfures, **caractérisé en ce qu'**on effectue l'hydrogénation dans un milieu alcoolique basique.

2. Procédé selon la revendication 1, **caractérisé en ce qu'**on prépare des arylmercaptans répondant à la formule (I) 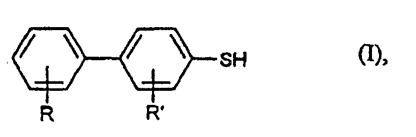 dans laquelle
R et R' représentent, indépendamment l'un de l'autre, un atome d'hydrogène, un groupe alkyle en C₁-C₆, un groupe alcoxy en C₁-C₆ ou un atome d'halogène,
à partir des diaryldisulfures correspondants répondant à la formule (II) 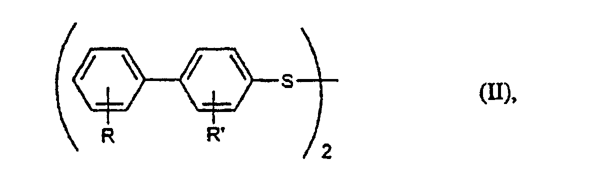 dans laquelle
R et R' ont la signification indiquée dans la formule (I).

3. Procédé selon les revendications 1 et 2, **caractérisé en ce qu'**on met en oeuvre, à titre de solvants pour la formation du milieu alcoolique, des alcools aliphatiques en C₁-C₅.

4. Procédé selon les revendications 1 à 3, **caractérisé en ce que** le milieu alcoolique contient de l'eau jusqu'à concurrence de 20 % en poids.

5. Procédé selon les revendications 1 à 4, **caractérisé en ce qu'**on met en oeuvre, à titre d'agent pour conférer un caractère basique au milieu alcoolique, des hydroxydes de métaux alcalins, des carbonates de métaux alcalins ou des alcoolates de métaux alcalins ou encore des composés correspondants de métaux alcalino-terreux.

6. Procédé selon les revendications 1 à 5, **caractérisé en ce qu'**on en oeuvre, pour 1 mole de diaryldisulfure, de 0,5 à 5 équivalents de base.

7. Procédé selon les revendications 1 à 6, **caractérisé en ce qu'**on met en oeuvre, à titre de catalyseur, des métaux du 8ème sous-groupe du système périodique des éléments.

8. Procédé selon les revendications 1 à 7, **caractérisé en ce qu'**on effectue l'hydrogénation catalytique à des températures de 20 à 200 °C et sous des pressions s'élevant jusqu'à 50 bar.

9. Procédé selon les revendications 1 à 8, **caractérisé en ce qu'**on effectue l'hydrogénation catalytique à une température de 40 à 150 °C et sous des pressions s'élevant jusqu'à 25 bar.

10. Procédé selon les revendications 1 à 9, **caractérisé en ce qu'**on traite le mélange réactionnel présent après la mise en oeuvre de l'hydrogénation catalytique de telle sorte qu'on le refroidit, on sépare le catalyseur, on ajoute de l'acide à la solution alcoolique présente de l'arylmercaptan qui a été préparé et on sépare ensuite par filtration le produit qui précipite et on le sèche.
